(19) Europäisches Patentamt
European Patent Office
Office européen des brevets

(11) Veröffentlichungsnummer: **0 097 614**
**B1**

(12) # EUROPÄISCHE PATENTSCHRIFT

(45) Veröffentlichungstag der Patentschrift: **04.11.87**

(51) Int. Cl.⁴: **C 07 D 207/22**, A 01 N 43/36

(21) Anmeldenummer: **83810242.4**

(22) Anmeldetag: **06.06.83**

(54) **Schädlingsbekämpfungsmittel.**

(30) Priorität: **11.06.82 CH 3639/82**
**11.06.82 CH 3641/82**
**26.08.82 CH 5079/82**

(43) Veröffentlichungstag der Anmeldung:
**04.01.84 Patentblatt 84/1**

(45) Bekanntmachung des Hinweises auf die Patenterteilung:
**04.11.87 Patentblatt 87/45**

(84) Benannte Vertragsstaaten:
**BE CH DE FR IT LI NL**

(56) Entgegenhaltungen:
**DE-A-1 937 665**
**GB-A-2 059 418**

(73) Patentinhaber: **CIBA- GEIGY AG, Klybeckstrasse 141, CH- 4002 Basel (CH)**

(72) Erfinder: **Gehret, Jean- Claude, Dr., Im Aeschfeld 12, CH- 4147 Aesch (CH)**
Erfinder: **Traber, Walter, Dr., Neueneichweg 12, CH- 4153 Reinach (CH)**

**EP 0 097 614 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue substituierte Phenylhydrazon- und Phenylhydrazin-Verbindungen, Verfahren zu ihrer Herstellung, Mittel, die diese Verbindungen als aktive Komponenten enthalten, und die Verwendung dieser Verbindungen und Mittel zur Bekämpfung von Schädlingen, Vertretern der Ordnung Acarina, darunter vor allem Ektoparasiten wie beispielsweise Milben und insbesondere Zecken.

In der deutschen Offenlegungsschrift Nr. 3 035 822 werden Phenylhydrazinpyrrolin-Verbindungen beschrieben und zur Bekämpfung von Milben vorgeschlagen. Diese Stoffe können jedoch die in der Praxis an sie gestellten Forderungen nur teilweise befriedigen.

Die neuen Verbindungen entsprechen der allgemeinen Formel Ia

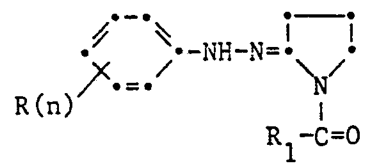

(Ia) ,

oder, in Abhängigkeit vom thermodynamischen Gleichgewicht, der allgemeinen Formel Ib oder der allgemeinen Formel Ic

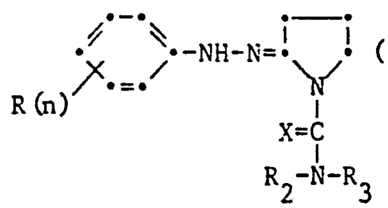

(Ib) ,

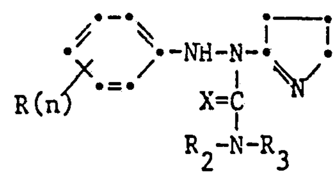

(Ic) ,

in welchen

R $C_1$-$C_4$-Alkyl oder Halogen

$R_1$ Wasserstoff gegebenenfalls durch Chlor, Fluor oder die Carboxylgruppe substituiertes $C_1$-$C_6$-Alkyl oder gegebenenfalls durch Chlor, Fluor oder die Carboxylgruppe substituiertes $C_2$-$C_6$-Alkenyl,

$R_2$ Wasserstoff oder Methyl,

$R_3$ Wasserstoff, $C_1$-$C_4$-Alkyl oder $C_2$-$C_4$-Alkenyl,

X Sauerstoff oder Schwefel darstellen, und

n 0 oder 1-5 bedeutet, wobei in Verbindungen, in denen n für 2 steht, auch ein R für Chlor und das andere R für Methyl stehen kann, und in welchen auch die Salze mit anorganischen oder organischen Säuren eingeschlossen sind.

Beispiele anorganischer Säuren sind Chlorwasserstoffsäure, Bromwasserstoffsäure, Jodwasserstoffsäure, Schwefelsäure, Phosphorsäure, phosphorige Säure, Salpetersäure.

Beispiele organischer Säuren sind Trifluoressigsäure, Trichloressigsäure, Oxalsäure, Bernsteinsäure, Maleinsäure, Milchsäure, Glycolsäure, Aconitsäure, Zitronensäure, Benzoesäure, Benzolsulfonsäure, Methansulfonsäure.

Von den Verbindungen der allgemeinen Formeln Ia, Ib und Ic sind diejenigen als bevorzugt anzusehen, in denen R Methyl und/oder Chlor und n 1 bzw. 2 bedeuten und $R_1$, $R_2$, $R_3$ und X die unter den Formeln Ia, Ib und Ic angegebenen Bedeutungen besitzen. Unter diesen sind wiederum solche Verbindungen der Formel Ia bevorzugt, worin, R(n) = 2,3-Dichlor bedeutet.

Die als Bedeutungen von R, $R_1$ und $R_3$ genannten Alkylreste umfassen Methyl, Äthyl und die Isomeren des Propyls, Butyls sowie für $R_1$ auch die des Pentyls und Hexyls. Unter den für $R_1$ und $R_3$ stehenden Alkenylresten sind Äthenyl sowie die Propenyle und die Butenyle und darüber hinaus für $R_1$ zusätzlich die Pentenyle und die Hexenyle zu verstehen.

Die Alkyl- und Alkenylreste können geradkettig oder verzweigt sein.

Die Verbindungen der Formel Ia können nach an sich bekannten Methoden (vgl. Houben-Weyl Bd. XI, Vol.2, S.3) wie folgt hergestellt werden:

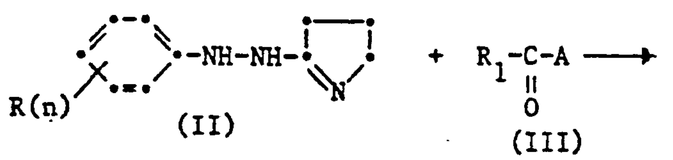

(II) (III) (Ia)

In den Formeln II und III haben die Symbole folgende Bedeutungen: A ist Halogen, vorzugsweise Chlor, oder die Gruppe -OC(O)$R_1$; R, $R_1$ und n besitzen die unter der Formel Ia angegebenen Bedeutungen.

Die Reaktion wird bei Temperaturen von 0° bis 100°C, vorzugsweise 10° bis 50°C, in Gegenwart eines

Lösungsmittels durchgeführt. Als Lösungsmittel gelten beispielsweise Alkane, Äther, Chlorkohlenwasserstoffe, aromatische Kohlenwasserstoffe, wie beispielsweise Benzol, Toluol oder die Xylole, oder organische Basen wie beispielsweise Pyridin, Triäthylamin oder N-Methylpyrrolidon, oder organische Säuren wie beispielsweise Essigsäure oder Chloressigsäure.

Die Verbindungen der Formeln Ib und Ic können, sofern $R_2$ Wasserstoff bedeuten soll, nach an sich bekannten Methoden (vgl. Houben-Weyl, Bd. VIII, S. 132 und S. 157) hergestellt werden, indem man Verbindungen der Formel IV

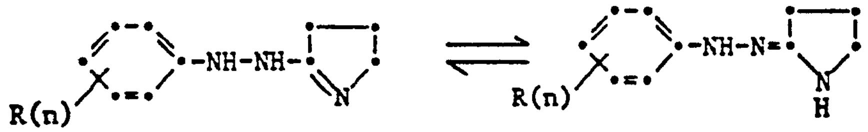
(IV)

mit Verbindungen der Formel IVa (Isocyanaten oder Isothiocyanaten)

$R_3N = C = X$ (IVa)

zur Reaktion bringt, wobei R, $R_3$, X und n die für die Formeln Ib und Ic angegebenen Bedeutungen haben.

Die Reaktion verläuft zwischen -30° und +100°C in Gegenwart eines Lösungsmittels. Zur gezielten Herstellung von Verbindungen der Formel Ic wird der Temperaturbereich zwischen -30° und +40°C, vorzugsweise -10° und +40°C, gewählt. Zur Herstellung von Verbindungen der Formel Ib wird der Temperaturbereich zwischen +55° bis +100°C, vorzugsweise +60° bis +80°C, gewählt. Im Übergangsbereich zwischen +40° und +55°C fallen beide Formen Ib und Ic, abhängig vom Einzelfall (Struktur, Reaktionszeit), als Gemisch an.

Als Lösungsmittel gelten beispielsweise Äther wie Diäthyläther, Tetrahydrofuran oder Dioxan, chlorierte Kohlenwasserstoffe wie Dichlormethan, Chloroform oder Tetrachlorkohlenwasserstoff, Aromaten wie Benzol, Toluol oder Xylole sowie weitere inerte Lösungsmittel wie Methyläthylketon oder Acetonitril. Verbindungen der Formel Ic lassen sich durch längeres Erhitzen, vorzugsweise in einem Lösungsmittel auf 55° bis 100°C, fast vollständig in die entsprechende Ib-Verbindung umwandeln.

In an sich bekannter Weise lassen sich auch Verbindungen der Formeln Ib und Ic durch Reaktion von Verbindungen der Formel IV mit einem Carbamoylchlorid der Formel IVb

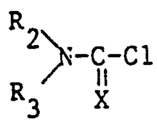
(IVb)

gewinnen, wobei R, $R_2$, $R_3$, X und n die vorgenannten Bedeutungen besitzen, $R_2$ und $R_3$ aber nicht Wasserstoff bedeuten. Für diese Reaktion gilt das Obengesagte sinngemäss. Die Reaktionstemperatur beträgt -30° bis +150°C. Die Reaktion wird in Gegenwart eines der oben genannten Lösungsmittel durchgeführt, wobei der Zusatz einer organischen Base wie beispielsweise Pyridin, Triäthylamin oder N-Methylpyrrolidin vorteilhaft ist, welche auch als alleiniges Lösungsmittel dienen kann.

Ferner ist es möglich, die Verbindungen der Formeln Ib und Ic herzustellen, indem man Amine der Formel $R_2R_3NH$ mit Phosgen zu den entsprechenden Carbamoylchloriden der Formel IVb umsetzt und diese dann in situ mit Verbindungen der Formel IV reagieren lässt, wobei die Reaktionstemperaturen und die verwendeten Reaktionsmedien denen der letztgenannten Methode entsprechen.

**Beispiel 1:**

**Herstellung von 2-[(N'-(2'-Chlor-4'-methylphenylhydrazono)]-1-chlorazetyl-pyrrolidin**

Zu 6,0 g (0,027 m) 2-[N'-(2'-Chlor-4'-methylphenylhydrazono)]-pyrrolin, gelöst in 80 ml Toluol, werden 4,6 g Chloressigsäureanhydrid, gelöst in 40 ml Toluol, langsam bei Raumtemperatur zugetropft. Die eintretende Reaktion verläuft schwach exotherm. Das Reaktionsgemisch wird dann 20 Stunden bei Raumtemperatur gerührt und anschliessend in einem Rotationsverdampfer eingeengt. Nach Abkühlung wird der Rückstand dann mit einem Essigsäureäthylester/Diäthyläther-Gemisch aufgeschlämmt und die Ausfällung als weisses Pulver abfiltriert.

Ausbeute: 3,2 g (= 39 % d.Th.). FP.: 134-136°C

**Beispiel 2:**

**Herstellung von 2-(N'-(2',3'-Dichlorphenylhydrazono)]-1-azetyl-pyrrolidin**

Zu 17,0 g (0,07 m) 2-[N'-(2',3'-Dichlorphenylhydrazino)]-pyrrolin, gelöst in 100 ml Dichlormethan, werden 8,2 g (0,08 m) Essigsäureanhydrid, gelöst in 20 ml Dichlormethan, langsam bei Raumtemperatur zugetropft. Die eintretende Reaktion verläuft exotherm. Das Reaktionsgemisch wird dann 20 Stunden bei ca. 40°C gerührt und anschliessend zuerst mit wässriger $NaHCO_3$-Lösung und dann mit NaCl-Lösung ausgeschüttelt. Nach Trocknen der organischen Phase wird diese eingeengt und der verbleibende Rückstand aus einem Essigsäureäthylester/Diäthyläther-Gemisch umkristallisiert. Das Produkt fällt in Form weisser Kristalle an.

Ausbeute: 13,0 g (= 65 % d.Th.), Fp.: 151-153°C.

Analog den vorstehend beschriebenen Beispielen werden folgende Verbindungen der Formel Ia hergestellt:

**Tabelle I:**

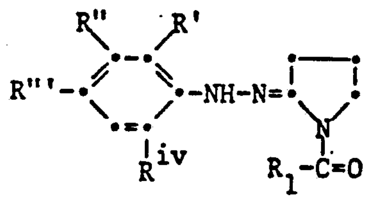

| Nr. | R' | R'' | R''' | $R^{iv}$ | $R_1$ | Physikal. Daten |
|---|---|---|---|---|---|---|
| 1 | Cl | H | $CH_3$ | H | $-CH_2Cl$ | Fp. 134-136°C |
| 2 | Cl | Cl | H | H | $-CH_3$ | Fp. 150-152°C |
| 3 | Cl | Cl | H | H | $-CH_2Cl$ | Fp. 149-151°C |
| 4 | Cl | Cl | H | H | $-CF_3$ | Fp. 145-147°C |
| 5 | Cl | Cl | H | H | $-C_2H_5$ | Fp. 138-139°C |
| 6 | Cl | Cl | H | H | $-C_3H_7$ | Fp. 98-100°C |
| 7 | Cl | Cl | H | H | $-CH(CH_3)_2$ | Fp. 151-153°C |
| 8 | Cl | Cl | H | H | $-CH=CH-COOH$ | Fp. 188°C Zers. |
| 9 | Cl | Cl | H | H | $-CH_2-CH_2-COOH$ | Fp. 176-177°C |
| 10 | Cl | H | H | H | $-CH_3$ | Fp. 128-129°C |
| 11 | H | Cl | H | H | $-CH_2Cl$ | Fp. 144-146°C |
| 12 | Cl | H | Cl | H | $-C_3H_7$ | Fp. 130-132°C |
| 13 | Cl | H | H | Cl | $-CH(CH_3)_2$ | $n^{D}_{24}$ 1,5637 |
| 14 | Cl | H | Cl | Cl | $-C_2H_5$ | Fp. 97-99°C |
| 15 | $CH_3$ | Cl | H | H | $-CH_2Cl$ | Fp. 150-151°C |
| 16 | Cl | H | $CH_3$ | H | $-CH(CH_3)_2$ | Fp. 109-113°C |
| 17 | Cl | Cl | H | H | H | Fp. 146-148°C |
| 18 | Cl | Cl | H | H | $n-C_5H_{11}$ | |
| 19 | $-CH_3$ | $-CH_3$ | H | H | $-CH_2Cl$ | |
| 20 | $-CH_3$ | $-CH_3$ | H | H | $-C_2H_5$ | |
| 21 | $-CH_3$ | H | Cl | H | $-CH_3$ | |
| 22 | $-CH_3$ | H | Cl | H | $-CH_2Cl$ | |
| 23 | $-CH_3$ | H | Cl | H | $i-C_3H_7$ | |
| 24 | $-CH_3$ | H | Cl | H | $n-C_6H_{13}$ | |
| 25 | $-CH_3$ | H | $-CH_3$ | H | $-CH_3$ | |
| 26 | $-CH_3$ | H | $-CH_3$ | H | $-CH_2Cl$ | |
| 27 | $-CH_3$ | H | $-CH_3$ | H | $n-C_3H_7$ | |
| 28 | Cl | Cl | H | H | $n-C_4H_9$ | Fp. 100-101°C |
| 29 | H | Cl | Cl | H | $-CH_3$ | Fp. 185-190°C |

**Tabelle II**

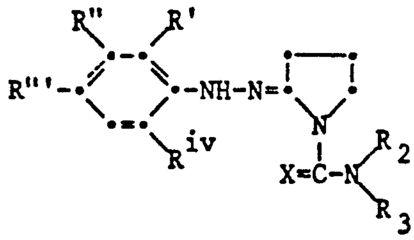

| Nr. | R′ | R″ | R‴ | $R^{iv}$ | $R_2$ | $R_3$ | X | Physikal. Daten [Fp. in °C] |
|---|---|---|---|---|---|---|---|---|
| 1 | Cl | Cl | H | H | H | $-CH_3$ | O | 148-150 |
| 2 | Cl | Cl | H | H | H | $-CH_3$ | S | 180-182 |
| 3 | Cl | Cl | H | H | H | $-CH(CH_3)_2$ | O | 140-150 Zs. |
| 4 | Cl | Cl | H | H | H | $-CH_2-CH=CH_2$ | O | 110-111 |
| 5 | Cl | H | H | H | $-CH_3$ | H | O | |
| 6 | Cl | H | H | H | H | $-CH_2CH=CH_2$ | S | |
| 7 | Cl | H | H | H | $-CH_3$ | H | S | |
| 8 | H | Cl | H | H | H | $-CH_2CH=CH_2$ | O | 116-118 |
| 9 | Cl | H | Cl | H | H | $-CH_2CH=CH_2$ | O | |
| 10 | Cl | H | H | Cl | H | $-CH_3$ | S | |
| 11 | Cl | H | H | Cl | H | $n-C_4H_9$ | O | |
| 12 | Cl | H | Cl | Cl | $-CH_3$ | H | O | |
| 13 | Cl | H | $-CH_3$ | H | H | $-CH(CH_3)_2$ | O | 109-113 |
| 14 | $-CH_3$ | Cl | H | H | H | $-C_2H_5$ | O | |
| 15 | Cl | Cl | H | H | H | $n-C_4H_9$ | O | 55-56 |
| 16 | Cl | Cl | H | H | H | $-CH_2CH=CH_2$ | S | 140-141 |
| 17 | $-CH_3$ | Cl | H | H | H | $-CH_2CH=CH_2$ | S | |
| 18 | $-CH_3$ | H | Cl | H | $-CH_3$ | H | S | |
| 19 | Cl | Cl | H | H | $-CH_3$ | $-CH_3$ | O | 97-99 |
| 20 | Cl | Cl | H | H | $-CH_3$ | $-CH_3$ | S | |
| 21 | Cl | $-CH_3$ | H | H | $-CH_3$ | $-CH_3$ | O | |
| 22 | Cl | $-CH_3$ | H | H | H | $-CH_3$ | S | |
| 23 | Cl | H | Cl | H | H | $i-C_3H_7$ | O | |
| 24 | Cl | Cl | H | H | H | $n-C_4H_9$ | S | 136-138 |
| 25 | H | Cl | Cl | H | H | $-CH_3$ | S | 171-172 |
| 26 | H | Cl | Cl | H | H | $-CH_3$ | O | 190-192 |

**Beispiel 3:**

**Herstellung von 2-[N′-(2,3-Dichlorphenyl-hydrazono)]-1-methylthiocarbamoyl-pyrrolidin**

Man lässt unter $N_2$-Atmosphäre bei 60°C 2,75 g (0,037 Mol) Methylisothiocyanat, gelöst in 60 ml Toluol, zu einer Lösung von 6,1 g (0,025 Mole) 2-[N′-(2,3-Dichlorphenylhydrazino)]-pyrrolin, gelöst in 80 ml Toluol während 1 Stunde zutropfen und rührt das Gemisch 20 Stunden bei dieser Temperatur. Nach dem Abkühlen im Eisbad kristallisieren 4 g Endprodukt (50 % d. Th.), die mit Diäthyläther gewaschen werden. Fp. 181-182°C. (Verb. Nr. 2/II).

**Beispiel 4**

**Herstellung von Verbindung 2/II durch thermische Umwandlung von Verbindung Nr. 2/III**

Man löst 0,5 g der Verbindung Nr. 2/III in 30 ml Toluol und erhitzt unter leichtem Rühren 24 Stunden auf 57-60°C. Danach engt man die Lösung ein und chromatographiert über Kieselgel mit Methylenchlorid als Laufmittel. Man erhält 0,47 g der Verbindung Nr. 2/II, Fp. 180-182°C.

Analog den vorstehend beschriebenen Beispielen 3 und 4 oder nach einem der weiter oben genannten Verfahren werden folgende Verbindungen hergestellt:

**Beispiel 5:**

**Herstellung von 2-[N'-(2,3-Dichlorphenyl)-N-(isopropyl-carbamoyl)-hydrazino]-pyrrolin**

Zu 7,3 g (0,03 m) 2-(N'-(2,3-Dichlorphenylhydrazino))-1-pyrrolin, gelöst in 100 ml Methylenchlorid, werden 2,8 g (0,033 m) Isopropylisocyanat, gelöst in 10 ml Methylenchlorid, bei 0° bis 5°C zugetropft. Das Reaktionsgemisch wird dann noch 1 Stunde bei ca. 5°C gerührt. Anschliessend wird das Lösungsmittel unter Vakuum abgedampft, der verbleibende weisse Rückstand in ca. 30 ml abs. Diäthyläther aufgeschlämmt und filtriert.

Nach Trocknung erhält man ein weisses Pulver.

Ausbeute: 8,5 g (86 % d.Th.), Fp.: 109-111°C (Verb. Nr. 3/III).

Analog dem vorstehend beschriebenen Beispiel werden folgende Verbindungen hergestellt:

**Tabelle III:**

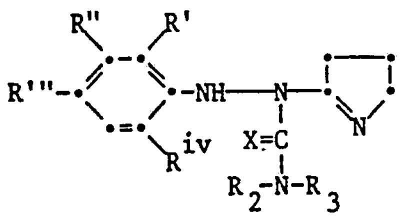

| Nr. | R' | R'' | R''' | $R^{iv}$ | $R_2$ | $R_3$ | X | Physikal. Daten (Fp.in °C) |
|---|---|---|---|---|---|---|---|---|
| 1 | Cl | Cl | H | H | H | $-CH_3$ | O | 109-110 |
| 2 | Cl | Cl | H | H | H | $-CH_3$ | S | 100-101 |
| 3 | Cl | Cl | H | H | H | $-CH(CH_3)_2$ | O | 109-111 |
| 4 | Cl | Cl | H | H | H | $-CH_2-CH=CH_2$ | O | 122-123 |
| 5 | Cl | H | H | H | $-CH_3$ | H | O | 97-99 |
| 6 | Cl | H | H | H | H | $-CH_2CH=CH_2$ | S | 96-97 |
| 7 | Cl | H | H | H | $-CH_3$ | H | S | 112-114 |
| 8 | H | Cl | H | H | H | $-CH_2CH=CH_2$ | O | 116-118 |
| 9 | Cl | H | Cl | H | H | $-CH_2CH=CH_2$ | O | 98-99 |
| 10 | Cl | H | H | Cl | H | $-CH_3$ | S | 117-120 |
| 11 | Cl | H | H | Cl | H | $n-C_4H_9$ | O | 94-96 |
| 12 | Cl | H | Cl | Cl | $-CH_3$ | H | O | 119-121 |
| 13 | Cl | H | $-CH_3$ | H | H | $-CH(CH_3)_2$ | O | 102-104 |
| 14 | $-CH_3$ | Cl | H | H | H | $-C_2H_5$ | O | 117-118 |
| 15 | Cl | Cl | H | H | H | $n-C_4H_9$ | O | 100-101 |
| 16 | Cl | Cl | H | H | H | $-CH_2CH=CH_2$ | S | 83-84 |
| 17 | $-CH_3$ | Cl | H | H | H | $-CH_2CH=CH_2$ | S | |
| 18 | $-CH_3$ | H | Cl | H | $-CH_3$ | H | S | |
| 19 | Cl | Cl | H | H | $-CH_3$ | $-CH_3$ | O | 83-88 |
| 20 | Cl | Cl | H | H | $-CH_3$ | $-CH_3$ | S | |
| 21 | Cl | $-CH_3$ | H | H | $-CH_3$ | $-CH_3$ | O | |
| 22 | Cl | $-CH_3$ | H | H | H | $-CH_3$ | S | |
| 23 | Cl | H | Cl | H | H | $i-C_3H_7$ | O | |
| 24 | Cl | Cl | H | H | H | $n-C_4H_9$ | S | 67-68 |
| 25 | H | Cl | Cl | H | H | $-CH_3$ | S | 94-95 |
| 26 | H | Cl | Cl | H | H | $-CH_3$ | O | 126-128 |

Die erfindungsgemässen Verbindungen der Formeln Ia, Ib und Ic oder die sie als aktive Komponente enthaltenden Mittel zeichnen sich in der Schädlingsbekämpfung durch besonders gute biologische Aktivität und ein günstiges Wirkungsspektrum aus. Darüber hinaus besitzen sie eine unerwartet hohe Stabilität. Als besonders geeignet zur Schädlingsbekämpfung erweisen sich folgende Verbindungen der Formel Ia:. 2-[N'-(2',3'-Dichlorphenyl-hydrazono)])-1-n-butyryl-pyrrolidin; 2-[N'-(2',3'-Dichlorphenyl-hydrazono)]-1-chloracetyl-pyrrolidin.

Die Aktivität der Wirkstoffe richter sich gegen Insekten und Vertreter der Ordnung Acarina, insbesondere gegen Zecken (Ixodidae), darunter vorzugsweise gegen die Spezies Rhipicephalus, Amblyomma und Boophilus, sowie gegen Milben wie beispielsweise Dermanyssus gallinae. Die Wirkungsbreite der erfindungsgemässen Verbindungen erfasst dabei al'e Entwicklungsstadien sowie die Ablage fertiler Eier. Die Wirksamkeit der erfindungsgemässen Verbindungen der Formel I richtet sich ferner gegen weitere Ektoparasiten wie Vertreter der Ordnungen Aphaniptera (z. B. blutsaugende Flöhe) und Phthiraptera (z. B. blutsaugende Läuse).

Die erhöhte Stabilität der erfindungsgemässen Verbindungen der Formeln Ia, Ib und Ic stellt eine Wirkungsdauer sicher, die zeitlich die Entwicklungszyklen mehrerer Parasitengenerationen umfasst, so dass je nach Anwendungsform, beispielsweise bei Nutztieren, eine einzige behandlung pro Saison ausreichend ist.

**Beispiel 6:**

**Test auf Wirkung gegen Zecken: Abtötungswirkung in verschiedenen Entwicklungsstadien**

Als Testobjekte werden Larven (jeweils ca. 50) oder Nymphen (jeweils ca. 25) der Zeckenarten Amblyomma hebraeum und Boophilus microplus verwendet. Die Testorganismen werden für kurze Zeit in wässrige Emulsionen bzw. Lösungen der Salze der zu untersuchenden Substanzen von bestimmter Konzentration getaucht. Die in den Teströhrchen befindlichen Emulsionen bzw. Lösungen werden dann mit Watte aufgenommen und die benetzten Testtiere in den so kontaminierten Röhrchen belassen. Die Auswertung erfolgt für Larven nach 3 Tagen und für Nymphen und Imagines nach 14 Tagen. Es wird die minimale Substanzkonzentration ermittelt, die die 100 %ige Abtötung bewirkt, ausgedrückt in ppm Wirksubstanz bezogen auf das Total von Emulsion oder Lösung.

Wirkstoffe der Tabellen I-III sind in folgenden Konzentrationsbereichen wirksam:

Larven: $<$ 0,1 - 10 ppm

Nymphen: $<$ 1 - 10 ppm

**Beispiel 7:**

**Test auf Wirkung gegen Zecken: Eiablagehemmung**

Als Testtiere werden vollgesogene Weibchen der Rinderzecke Boophilus microplus verwendet. Pro Konzentration werden je 10 Zecken eines OP-resistenten Stammes (z. B. Biarra-Stamm) und eines normal empfindlichen Stammes (z. B. Yeerongpilly-Stamm) behandelt. Die Zecken werden auf mit Doppelklebeband bezogenen Platten fixiert und dann entweder mit wässrigen Emulsionen bzw. mit Lösungen der Salze der zu untersuchenden Verbindungen benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird die totale Hemmung der Ablage von fertilen Eiern ermittelt.

Die Hemmwirkung der Substanzen wird ausgedrückt als minmale Substanzkonzentration in ppm bei 100%iger Wirkung gegen normal sensible und resistente adulte weibliche Zecken. Wirkstoffe der Tabellen I-III sind im Konzentrationsbereich von $<$ 16-125 ppm voll wirksam.

**Beispiel 8:**

**Test auf pflanzenparasitäre Insekten**

Baumwollpflanzen werden mit einer Versuchslösung, enthaltend 50 und 100 ppm der zu prüfenden Verbindung, besprüht.

Nach dem Antrocknen des feuchten Belages werden die Baumwollpflanzen mit Spodoptera littoralis-Larven $L_3$ besetzt. Der Versuch wird bei 24°C und 60 % relativer Luftfeuchtigkeit durchgeführt.

Wirkstoffe der Tabellen I-III zeigen folgende Frassgift-Wirkung gegen Spodoptera-Larven:

70-100 % Abtötung bei 50 oder 100 ppm Wirkstoffkonzentration.

Ähnliche Resultate, wie in den vorstehenden Beispiel 6 bis 8 angegeben, konnten mit Wirkstoffen aus den Tabellen I-III auch bei längerer Anwendungszeit unter Praxisbedingungen ohne Durchführung zusätzlicher pH-Wert-stabilisierender Massnahmen in den praxisbezogenen Anwendungsformen erzielt werden.

Zur Bekämpfung der Schädlinge werden die erfindungsgemässen Verbindungen der Formeln Ia, Ib oder Ic sowohl allein als auch in Form von Mitteln eingesetzt, welche noch geeignete Trägerstoffe oder Zuschlagstoffe oder Gemische solcher Stoffe enthalten. Geeignete Träger- und Formulierungshilfsstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs- oder Bindemitteln.

Zur Applikation können die Verbindungen der Formeln Ia, Ib oder Ic zu Stäubemitteln, Emulsionskonzentraten, Granulaten, Dispersionen, Sprays, zu Lösungen oder Aufschlämmungen in üblicher Formulierung die in der Applikationstechnik zum Allgemeinwissen gehören, verarbeitet werden.

Die Herstellung der erfindungsgemässen Mittel erfolgt in an sich bekannter Weise durch inniges Vermischen und/oder Vermahlen von Wirkstoffen der Formeln Ia, Ib oder Ic mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate Umhüllungsgranulate, Imprägnierungsgranulate und Homogengranulate).

Flüssige Aufarbeitungsformen:

a) in Wasser dispergierbare Wirkstoffkonzentrate: Spritzpulver (wettable Powder), Pasten, Emulsionen;

b) Lösungen: Aufguss, Sprühmittel.

Der Gehalt an Wirkstoff in den vorstehend beschriebenen Applikationsformen liegt vorzugsweise zwischen 0,1 und 95,0 Gew.%.

**Beispiel 9:**

**Emulsionskonzentrat**

20 Gew.-Teile Wirkstoff werden in

70 Gew.-Teilen Xylol gelöst und mit

10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

**Beispiel 10:**

**Emulsionskonzentrat**

5 bis max. 30 Gew.-Teile Wirkstoff werden unter Rühren bei Zimmertemperatur in

30 Gew.-Teilen Dibutylphthalat

10 Gew.-Teilen Solvent (niederviskoses hocharomatisches Erdöldestillat)

15 bis 35 Gew.-Teilen Dutrex 238 FC (viskoses hocharomatisches Erdöldestillat) gelöst und mit

10 Gew.-Teilen eines Emulgatorgemisches, bestehend aus Ricinusöl-Polyglykoläther und dem Calciumsalz der Dodecylbenzolsulfonsäure, versetzt. Das so erhaltene Emulsionskonzentrat gibt in Wasser milchige Emulsionen.

**Beispiel 11:**

**Spritzpulver**

5 bis 30 Gew.-Teile Wirkstoff werden in einer Mischapparatur mit

5 Gew.-Teilen eines aufsaugenden Trägermaterials (Kieselsäure K 320 oder Wessalon S) und

55 bis 80 Gew.-Teilen eines Trägermaterials (Bolus alba oder Kaolin (B 24) und einem Dispergiermittelgemisch, bestehend aus

5 Gew.-Teilen eines Na-Laurylsulfonats und

5 Gew.-Teilen eines Alkyl-aryl-polyglykoläthers, intensiv vermischt. Diese Mischung wird auf einer Stift- oder Luftstrahlmühle bis auf 5-15 μm gemahlen. Das so erhaltene Spritzpulver gibt in Wasser eine gute Suspension.

**Beispiel 12:**

**Stäubemittel**

5 Gew.-Teile feingemahlener Wirkstoff werden mit
2 Gew.-Teilen einer gefällten Kieselsäure und
93 Gew.-Teilen Talk intensiv gemischt.

**Beispiel 13:**

**Aufguss-Lösung** ("Pour on")

| | |
|---|---|
| Wirksubstanz | 30,0 g |
| Natrium-dioctylsulfosuccinat | 3,0 g |
| Benzylalkohol | 48,0 g |
| Erdnussöl | 19,8 g |
| 100 | ,8 g = 100 ml |

Die Wirksubstanz wird in dem Benzylalkohol unter Rühren, eventuell auch unter leichtem Erwärmen, gelöst. Zu der Lösung wird das Natriumdioctylsulfosuccinat und das Erdnussöl gegeben und unter Erwärmen und gründlichem Durchmischen gelöst.

**Beispiel 14:**

**Aufguss-Lösung** ("Pour on")

| | |
|---|---|
| Wirkstubstanz | 30,00 g |
| Natrium-Dioctylsulfosuccinat | 3,00 g |
| Benzylalkohol | 35,46 g |
| Äthylenglykol-monomethyläther | 35,46 g |
| | 103,92 g = 100 ml |

Die Wirksubstanz wird in dem grössten Teil des Gemisches der beiden Lösungsmittel unter kräftigem Rühren gelöst. Anschliessend wird das Natrium-dioctylsulfosuccinat, eventuell unter Erwärmen, gelöst und schliesslich mit dem restlichen Lösungsmittelgemisch aufgefüllt.

**Patentansprüche**

1. Substituierte Phenylhydrazon- und Phenylhydrazin-Verbindungen der allgemeinen Formel Ia

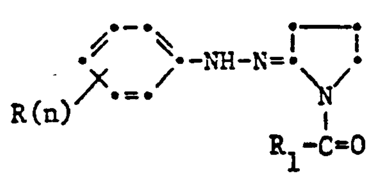

(Ia) ,

oder, in Abhängigkeit vom thermodynamischen Gleichgewicht, der allgemeinen Formel Ib oder Ic

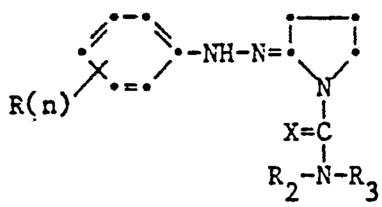 (Ib) , 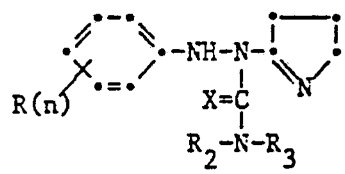 (Ic) ,

in welchen
R $C_1$-$C_4$-Alkyl oder Halogen,
$R_1$ Wasserstoff, gegebenenfalls durch Chlor, Fluor oder die Carboxylgruppe substituiertes $C_1$-$C_6$-Alkyl oder gegebenenfalls durch Chlor, Fluor oder die Carboxylgruppe substituiertes $C_2$-$C_6$-Alkenyl,
$R_2$ Wasserstoff oder Methyl,
$R_3$ Wasserstoff, $C_1$-$C_4$-Alkyl oder $C_2$-$C_4$-Alkenyl,
X Sauerstoff oder Schwefel darstellen, und
n 0 oder 1-5 bedeutet, wobei in Verbindungen, in denen n für 2 steht, auch ein R für Chlor und das andere R für Methyl stehen kann, unter Einschluss ihrer Salze mit anorganischen und organischen Säuren.

2. Verbindungen der Formeln Ia, Ib oder Ic gemäss Anspruch 1, in welchen R Methyl und/oder Chlor und n 1 bzw. 2 bedeuten und $R_1$, $R_2$, $R_3$ und X die in Anspruch 1 angegebenen Bedeutungen besitzen.

3. Verbindungen der Formel Ia gemäss Anspruch 2, in welchen R(n) 2,3-Dichlor bedeutet.

4. Verbindung 2-[N'-(2',3'-Dichlorphenyl-hydrazono)]-1-n-butyryl-pyrrolidin gemäss Anspruch 3.

5. Verbindung 2-[N'-(2',3'-Dichlorphenyl-hydrazono)]-1-chloracetyl-pyrrolidin gemäss Anspruch 3.

6. Verfahren zur Herstellung von Verbindungen der Formel Ia gemäss Anspruch 1, dadurch gekennzeichnet, dass man Verbindungen der Formel II

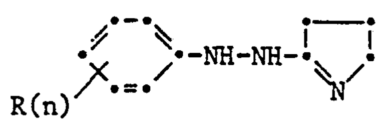 (II)

mit Verbindungen der Formel III

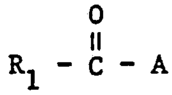 (III),

in welchen
R $C_1$-$C_4$-Alkyl oder Halogen,
$R_1$ Wasserstoff, $C_1$-$C_6$-Alkyl, durch Chlor, Fluor oder Carboxyl substituiertes $C_1$-$C_6$-Alkyl, $C_2$-$C_6$-Alkenyl oder durch Chlor, Fluor oder die Carboxylgruppe substituiertes $C_2$-$C_6$-Alkenyl,
A Halogen oder die Gruppe -OC(O)$R_1$ und
n 0 oder 1-5 bedeuten, wobei in Verbindungen, in denen n für 2 steht, auch ein R für Chlor und das andere R für Methyl stehen kann, in einem Lösungsmittelmedium bei Temperaturen von 0° bis 100°C umsetzt.

7. Verfahren zur Herstellung von Verbindungen der Formeln Ib oder Ic gemäss Anspruch 1 mit $R_2$= Wasserstoff, dadurch gekennzeichnet, dass man Verbindungen der Formel IV

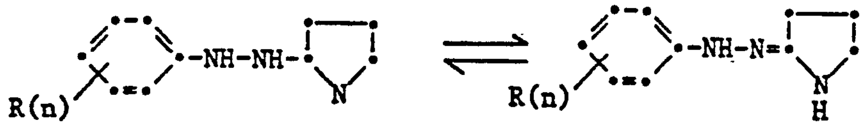 (IV) .

mit Verbindungen der Formel IVa

$R_3N=C=X$ (IVa)

in welchen
R $C_1$-$C_4$-Alkyl oder Halogen,
$R_1$ Wasserstoff, $C_1$-$C_4$-Alkyl oder $C_2$-$C_4$-Alkenyl,
X Sauerstoff oder Schwefel darstellen und
n 0 oder 1-5 bedeutet, wobei in Verbindungen, in denen n für 2 steht, auch ein R für Chlor und das andere R für Methyl stehen kann, im Temperaturbereich von -30° bis +100°C in Gegenwart eines Lösungsmittels zur Reaktion bringt, wobei zur gezielten Herstellung von Derivaten der Formel Ic im Temperaturbereich von -30° bis +40°C und von Derivaten der Formel Ib im Temperaturbereich von +55° bis +100°C gearbeitet wird.

8. Verfahren zur Herstellung von Verbindungen der Formeln Ib oder Ic gemäss Anspruch 1, dadurch gekennzeichnet, dass man Verbindungen der Formel IV

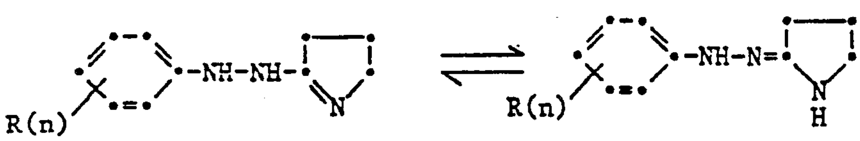 (IV) .

mit Verbindungen der Formel IVb

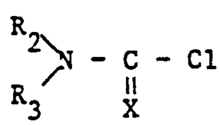 (IVb) ,

in welchen
R $C_1$-$C_4$-Alkyl oder Halogen
$R_2$ Methyl,
$R_3$ $C_1$-$C_4$-Alkyl oder $C_2$-$C_4$-Alkenyl,
X Sauerstoff oder Schwefel darstellen und
n 0 oder 1-5 bedeutet, wobei in Verbindungen, in denen n für 2 steht, auch ein R für Chlor und das andere R für Methyl stehen kann,
im Temperaturbereich von -30° bis +150°C in Gegenwart eines Lösungsmittels umsetzt.

9. Schädlingsbekämpfungsmittel, dadurch gekennzeichnet, dass es als aktive Komponente mindestens eine Verbindung der allgemeinen Formeln Ia, Ib oder Ic gemäss Anspruch 1 neben inerten Träger- und Formulierungshilfsstoffen enthält.

10. Schädlingsbekämpfungsmittel gemäss Anspruch 9, dadurch gekennzeichnet, dass es als aktive Komponente mindestens eine Verbindung gemäss den Ansprüchen 2 und 3 enthält.

11. Schädlingsbekämpfungsmittel gemäss Anspruch 10, dadurch gekennzeichnet, dass es als aktive Komponente mindestens eine Verbindung gemäss den Ansprüchen 4 und 5 enthält.

12. Verfahren zur Bekämpfung von Schädlingen auf oder in ihren Lebensräumen mit Ausnahme menschlicher oder tierischer Körper, dadurch gekennzeichnet, dass eine Verbindung der allgemeinen Formel Ia, Ib oder Ic gemäss Anspruch 1 verwendet wird.

13. Verfahren gemäss Anspruch 12 zur Bekämpfung von Insekten.

14. Verfahren gemäss Anspruch 12 zur Bekämpfung von Ektoparasiten.

15. Verfahren gemäss Anspruch 14 zur Bekämpfung von Acarina.

16. Verfahren gemäss Anspruch 15 zur Bekämpfung von Zecken.

17. Verbindung der Formel I gemäß Anspruch 1 zur Verwendung als antiparasitäres Mittel gegen Schädlinge der Gruppe bestehend aus Insekten und Vertretern der Ordnung Acarina.

**Claims**

1. Substituted phenylhydrazone and phenylhydrazine compounds of the general formula Ia

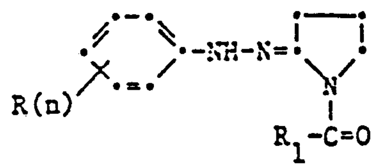

(Ia),

or, depending on the thermodynamic equilibrium, of the general formula Ib or Ic

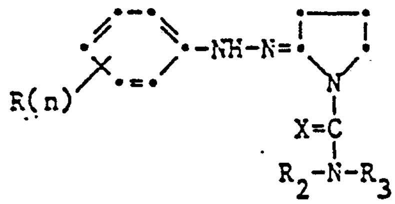

(Ib),

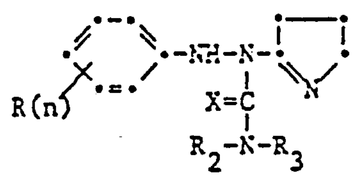

(Ic)

wherein

R is $C_1$-$C_4$-alkyl or halogen,

$R_1$ is hydrogen, $C_1$-$C_6$-alkyl which is unsubstituted or substituted by chlorine, fluorine or the carboxyl group, or it is $C_2$-$C_6$-alkenyl which is unsubstituted or substituted by chlorine, fluorine or the carboxyl group,

$R_2$ is hydrogen or methyl,

$R_3$ is hydrogen, $C_1$-$C_4$-alkyl or $C_2$-$C_4$-alkenyl,

X is oxygen or sulfur, and

n is 0 or 1-5, and in compounds in which n is 2, also one R may be chlorine and the other R may be methyl, including the salts thereof with inorganic and organic acids.

2. Compounds of the formula Ia, Ib or Ic according to Claim 1, wherein R is methyl and/or chlorine, and n is 1 or 2, and $R_1$, $R_2$, $R_3$ and X are as defined in Claim 1.

3. Compounds of the formula Ia according to Claim 2, wherein R(n) is 2,3-dichloro.

4. A compound: 2-[N'-(2',3'-dichlorophenyl-hydrazono)]-1-n-butyryl-pyrrolidine according to Claim 3.

5. A compound: 2-[N'-(2',3'-dichlorophenyl-hydrazono)]-1-chloroacetyl-pyrrolidine according to Claim 3.

6. A process for producing compounds of the formula Ia according to Claim 1, characterised in that compounds of the formula II

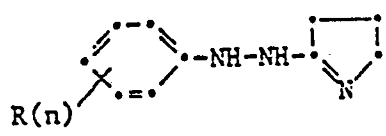

(II)

are reacted with compounds of the formula III

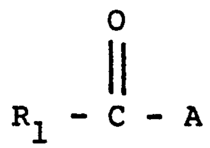

(III)

wherein
R is $C_1$-$C_4$-alkyl or halogen,
$R_1$ is hydrogen, $C_1$-$C_6$-alkyl, $C_1$-$C_6$-alkyl substituted by chlorine, fluorine or carboxyl, or it is $C_2$-$C_6$-alkenyl, or $C_2$-$C_6$-alkenyl substituted by chlorine, fluorine or the carboxyl group,
A is halogen or the group $-OC(O)R_1$, and
n is 0 or 1-5, and in compounds in which n is 2, also one R may be chlorine and the other R may be methyl,
in a solvent medium at temperatures of 0° to 100°C.

7. A process for producing compounds of the formula Ib or Ic according to Claim 1 wherein $R_2$ is hydrogen, characterised in that compounds of the formula IV

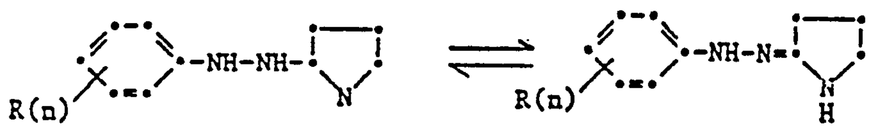

(IV)

are reacted with compounds of the formula IVa

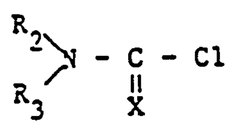

(IVb)

wherein
R is $C_1$-$C_4$-alkyl or halogen,
$R_3$ is hydrogen, $C_1$-$C_4$-alkyl or $C_2$-$C_4$-alkenyl,
X is oxygen or sulfur, and
n is 0 or 1-5, and in compounds in which n is 2, also one R may be chlorine and the other R may be methyl,
in the temperature range of -30° to +100°C in the presence of a solvent, the reaction being performed in the temperature range of -30° to +40°C for specifically producing derivatives of the formula Ic, and in the temperature range of +55° to +100°C for specifically producing derivatives of the formula Ib.

8. A process for producing compounds of the formula Ib or Ic according to Claim 1, characterised in that compounds of the formula IV

$$R_3N{=}C{=}X \qquad \text{(IVa)}$$

are reacted with compounds of the formula IVb

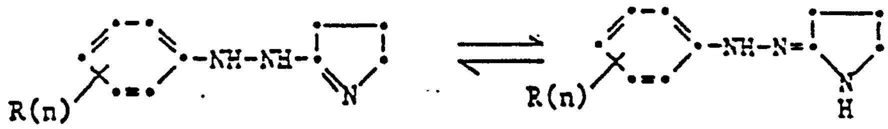

(IV)

wherein
R is $C_1$-$C_4$-alkyl or halogen,
$R_2$ is methyl,
$R_3$ is $C_1$-$C_4$-alkyl or $C_2$-$C_4$-alkenyl,
X is oxygen or sulfur, and

n is 0 or 1-5, and in compounds in which n is 2, also one R may be chlorine and the other R may be methyl, in the temperature range of -30° to +150° C in the presence of a solvent.

9. A pesticidal composition, characterised in that it contains as active ingredient at least one compound of the general formula Ia, Ib or Ic according to Claim 1, together with inert carriers and formulation auxiliaries.

10. A pesticidal composition according to Claim 9, characterised in that it contains as active ingredient at least one compound according to Claims 2 and 3.

11. A pesticidal composition according to Claim 10, characterised in that it contains as active ingredient at least one compound according to Claims 4 and 5.

12. A process for controlling pests on or in their living environments with the exception of the human or animal body, characterised in that a compound of the general formula Ia, Ib or Ic according to Claim 1 is used.

13. A process according to Claim 12 for controlling insects.

14. A process according to Claim 12 for controlling ectoparasites.

15. A process according to Claim 14 for controlling Acarina.

16. A process according to Claim 15 for controlling ticks.

17. A compound of the formula I according to Claim 1 for use as an anti-parasitic agent against pests from the group consisting of insects and members of the order Acarina.

**Revendications**

1. Dérivés substitués de la phénylhydrazone et de la phénylhydrazine répondant à la formule générale Ia

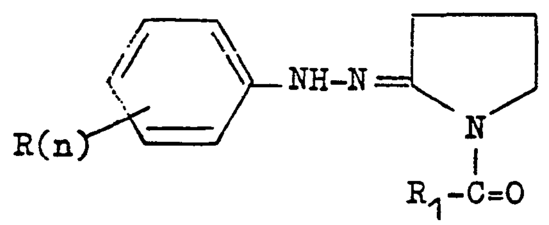

(Ia),

ou bien, selon l'équilibre thermodynamique, à la formule générale Ib ou Ic

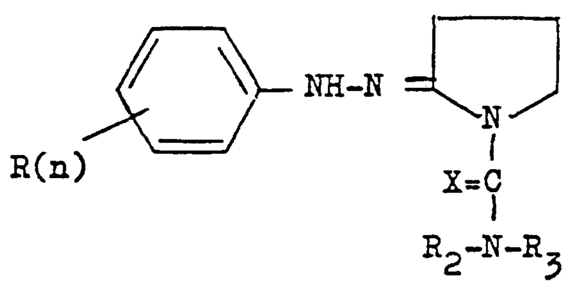

(Ib) ,

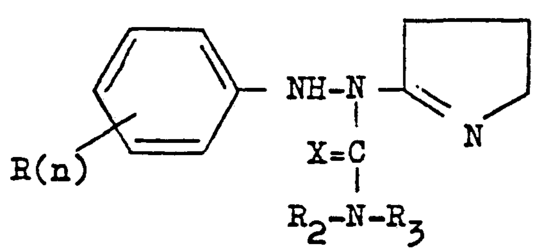

(Ic),

dans lesquelles

R représente un groupe alkyle en C1-C4 ou un halogène,

$R_1$ représente l'hydrogène, un groupe alkyle en C1-C6 éventuellement substitué par le chlore, le fluor ou le groupe carboxyle ou un groupe alcényle en C2-C6 éventuellement substitué par le chlore, le fluor ou le groupe carboxyle,

$R_2$ représente l'hydrogène ou un groupe méthyle,

$R_3$ représente l'hydrogène, un groupe alkyle en C1-C4 ou alcényle en C2-C4,

X représente l'oxygène ou le soufre, et

n est égal à 0 ou a une valeur de 1 à 5, étant spécifié que dans les composés pour lesquels n est égal à 2, l'un des symboles R peut également représenter le chlore et l'autre un groupe méthyle,

y compris leurs sels d'acides minéraux et organiques.

2. Composés de formules Ia, Ib ou Ic selon la revendication 1, dans lesquels R représente un groupe méthyle et/ou le chlore et n est égal à 1 ou 2, $R_1$, $R_2$, $R_3$ et X ayant les significations indiquées dans la revendication 1.

3. Composés de formule Ia selon la revendication 2, dans lesquels R(n) représente un groupe 2,3-dichloro.

4. Composé selon la revendication 3 : la 2-[N'-(2',3'-dichlorophényl-hydrazono)]-1-n-butyryl-pyrrolidine.

5. Composé selon la revendication 3 : la 2-[N'-(2',3'-dichlorophényl-hydrazono)]-1-chloracétyl-pyrrolidine.

6. Procédé de préparation des composés de formule Ia selon la revendication 1, caractérisé en ce que l'on fait réagir dans un milieu solvant à des températures de 0 à 100° C des composés de formule II

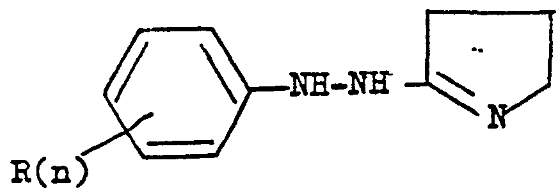

(II)

avec des composés de formule III

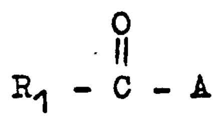

(III),

dans lesquelles

R représente un groupe alkyle en C1-C4 ou un halogène,

$R_1$ représente l'hydrogène, un groupe alkyle en C1-C6, un groupe alkyle en C1-C6 substitué par le chlore, le fluor ou un groupe carboxyle, un groupe alcényle en C2-C6 ou un groupe alcényle en C2-C6 substitué par le chlore, le fluor ou le groupe carboxyle,

A représente un halogène ou le groupe $-OC(O)R_1$, et

n est égal à 0 ou a une valeur de 1 à 5, étant spécifié que dans les composés pour lesquels n est égal à 2, l'un des symboles R peut représenter le chlore et l'autre un groupe méthyle.

7. Procédé de préparation des composés de formule Ib ou Ic selon la revendication 1, dans lesquels R représente l'hydrogène, caractérisé en ce que l'on fait réagir des composés de formule IV

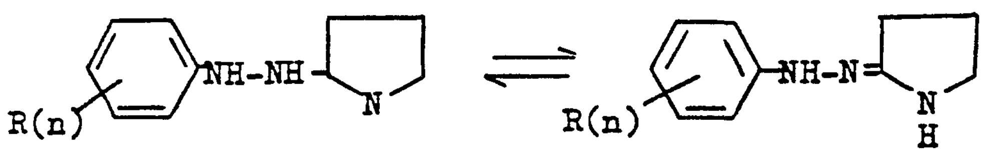

(IV)

avec des composés de formule IVa

$R_3N=C=X$ (IVa)

dans lesquelles

R représente un groupe alkyle en C1-C4 ou un halogène,

$R_3$ représente l'hydrogène, un groupe alkyle en C1-C4 ou alcényle en C2-C4,

X représente l'oxygène ou le soufre, et

n est égal à 0 ou a une valeur de 1 à 5, étant spécifié que dans les composés pour lesquels n est égal à 2, l'un des symboles R peut également représenter le chlore et l'autre un groupe méthyle,

dans l'intervalle de température de -30 à +100° C, en présence d'un solvant, en opérant dans l'intervalle de température de -30 à +40° C lorsqu'on veut obtenir des dérivés de formule Ic et dans l'intervalle de température de +55 à +100° C lorsqu'on veut obtenir des dérivés de formule Ib.

8. Procédé de préparation des composés de formule Ib ou Ic selon la revendication 1, caractérisé en ce que l'on fait réagir des composés de formule IV

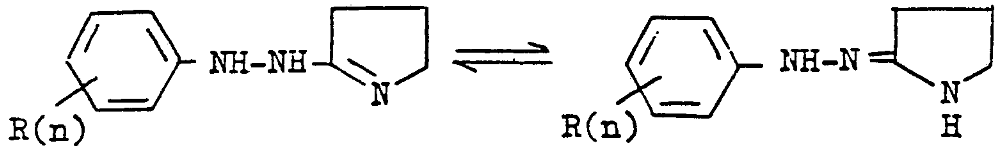

(IV)

avec des composés de formule IVb

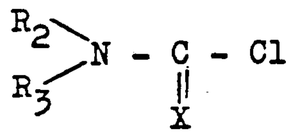

(IVb),

dans lesquelles

R représente un groupe alkyle en C1-C4 ou un halogène,

$R_2$ représente le groupe méthyle,

$R_3$ représente un groupe alkyle en C1-C4 ou alcényle en C2-C4,

X représente l'oxygène ou le soufre, et

n est égal à 0 ou a une valeur de 1 à 5, étant spécifié que dans les composés pour lesquels n est égal à 2, l'un des symboles R peut également représenter le chlore et l'autre un groupe méthyle,

dans l'intervalle de température de -30 à +150° C, en présence d'un solvant.

9. Produit pesticide caractérisé en ce qu'il contient en tant que composant actif au moins un composé de formule générale Ia, Ib ou Ic selon la revendication 1 avec des véhicules et produits auxiliaires de formulation inertes.

10. Produit pesticide selon la revendication 9, caractérisé en ce qu'il contient en tant que composant actif au moins un composé selon les revendications 2 et 3.

11. Produit pesticide selon la revendication 10, caractérisé en ce qu'il contient en tant que composant actif au moins un composé selon les revendications 4 et 5.

12. Procédé pour lutter contre les parasites sur leur espace vital ou dans leur espace vital, à l'exception du corps humain ou animal, caractérisé en ce que l'on utilise un composé de formule générale Ia, Ib ou Ic selon la revendication 1.

13. Procédé selon la revendication 12, pour la lutte contre les insectes.

14. Procédé selon la revendication 12, pour la lutte contre les ectoparasites.

15. Produit selon la revendication 14, pour la lutte contre les acariens.

16. Procédé selon la revendication 15, pour la lutte contre les tiques.

17. Composé de formule I selon la revendication 1, pour l'utilisation en tant que produit antiparasitaire contre les parasites du groupe consistant en les insectes et les représentants de l'ordre des acariens.